# EUROPEAN PATENT APPLICATION

(11) **EP 4 495 939 A1**
(43) Date of publication of application: **22.01.2025**
(21) Application number: 23186742.5
(22) Date of filing: 20.07.2023
(51) Int. Cl.: G16H 15/00, G16H 30/40

(54) **METHODS AND SYSTEMS FOR PROVIDING A MEDICAL VIDEO REPORT**

(71) Applicant: Siemens Healthineers AG, 91301 Forchheim (DE)
(72) Inventor: Huber, Martin, 91080 Uttenreuth (DE); Kohl, Gerhard, 91077 Neunkirchen am Brand (DE); Rusitska, Michael, 91080 Utternreuth (DE)
(74) Representative: Siemens Healthineers Patent Attorneys

(57) **Abstract**

Method and systems for providing a medical video report (MVR) are disclosed. Specifically, it is proposed to extract a context information (CI) pertaining to a medical image study (MIS) and to select (S30) a template video script (TVS) from a plurality of candidate template video scripts (CVS) using the context information (CI). The candidate template video scripts (CVS) respectively encode instructions for image processing steps of a medical image study (MIS) in order to generate a video visualizing a medical diagnosis to a human recipient (HR). Then, the selected template video script (TVS) may be executed so as to generate the medical video report (MVR) .

## Description

Embodiments of the invention concern methods and systems for providing a medical video report. In particular, embodiments of the invention concern the automated generation of a medical video report corresponding to a medical image study. Further, embodiments of the invention concern the generation of a medical report for a layperson such as a patient.

Medical text reports are the principal instrument in medicine to relay medical diagnosis to the stakeholders of a case. Medical reports are not only exchanged between physicians but also provided to the patient. The generation of medical text reports is subjected to time and legal constraints. As a result, medical text reports are generally not an instructive read. What is more, the style of a medical report may vary considerably between different physicians.

As a consequence, the majority of patients is struggling to understand medical text reports. Often, medical reports use terms and expressions medical nonprofessionals are not familiar with and cannot make sense of. Consequently, patients feel insecure and left alone when confronted with a medical report which is detrimental for the patient engagement.

Even many referring physicians may sometimes face difficulties in interpreting the medical reports they receive from expert physicians. What is more, treating physicians may be confronted with questions from their patients regarding the medical reports and have to invest additional time in explaining the report and clarifying misunderstandings at the side of the patient.

To improve the situation, it has been proposed to use radiology report templates in simplified language. However, these are currently only available for a few standardized treatments. Moreover, preparing such a simplified version in addition to the required medico legal text report constitutes an extra effort for the physicians for which the tight clinical workflows usually do not provide sufficient time.

Accordingly, it is an object of embodiments of the invention to provide improved methods and systems which enable an improved way of communicating a clinical outcome to a recipient. In particular, it is an object of embodiments of the invention to enable the creation of an artifact which allows the recipient to better understand the medical background and implications of a particular outcome. Moreover, it is an object to enable generating this artifact without significantly increasing the workload of the diagnosing physician/radiologist and with minimal interference with the reading and reporting workflow.

This object is solved by a method for providing a medical video report, corresponding systems, corresponding computer-program products, and computer-readable storage media according to the main claims. Alternative and/or preferred embodiments are object of the dependent claims.

In the following, the technical solution according to the present invention is described with respect to the claimed apparatuses as well as with respect to the claimed methods. Features, advantages, or alternative embodiments described herein can likewise be assigned to other claimed objects and vice versa. In other words, claims addressing the inventive method can be improved by features described or claimed with respect to the apparatuses. In this case, e.g., functional features of the method are embodied by objective units or elements of the apparatus.

According to an aspect, a computer-implemented method for providing a medical video report is provided. The method comprises several steps. One step is directed to receive a medical image study of a patient. Another step is directed to determine, based on the medical image study, a context information pertaining to the medical image study. Another step is directed to select, based on the context information, a template video script from a plurality of candidate template video scripts, each candidate template video script encoding instructions for image processing steps of a medical image study in order to generate a video relating to the medical image study (or: a video visualizing information relating to the medical image study) to a human recipient. Another step is directed to execute the selected template video script on the medical image study so as to generate the medical video report. Another step is directed to provide the medical video report.

The medical image study may comprise three-dimensional data sets providing three dimensions in space or two dimensions in space and one dimension in time, two-dimensional data sets providing two dimensions in space, and/or four-dimensional data sets providing three dimensions in space and one dimension in time.

The medical image study may depict a body part of a patient in the sense that it contains three-dimensional image data of the patient's body part. The medical image study may be representative of an image volume. The patient's body part may be comprised in the image volume.

The medical image study comprises image data, for example, in the form of a two- or three-dimensional array of pixels or voxels. Such arrays of pixels or voxels may be representative of intensity, absorption, or other parameters as a function of three-dimensional position, and may, for example, be obtained by suitable processing of measurement signals obtained by a medical imaging modality.

A medical imaging modality corresponds to a system used to generate or produce medical image data. For example, a medical imaging modality may be a computed tomography system (CT system), a magnetic resonance system (MR system), an angiography (or C-arm X-ray) system, a positron-emission tomography system (PET system), an ultrasound imaging system or the like. Specifically, ccomputed tomography is a widely used imaging method and makes use of "hard" X-rays produced and detected by a specially rotating instrument. The resulting attenuation data (also referred to as raw data) is presented by a computed analytic software producing detailed images of the internal structure of the patient's body parts. The produced sets of images are called CT-scans which may constitute multiple series of sequential images to present the internal anatomical structures in cross sections perpendicular to the axis of the human body. Magnetic Resonance Imaging (MRI), to provide another example, is an advanced medical imaging technique which makes use of the effect magnetic field impacts on movements of protons. In MRI machines, the detectors are antennas, and the signals are analyzed by a computer creating detailed images of the internal structures in any section of the human body.

The medical image study may be stored in a standard image format such as the Digital Imaging and Communications in Medicine (DICOM) format and in a memory or computer storage system such as a Picture Archiving and Communication System (PACS), a Radiology Information System (RIS), a Vendor Neutral Archive (VNA), an Electronic Health Record (HER) storge or database, and the like. Whenever DICOM is mentioned herein, it shall be understood that this refers to the "Digital Imaging and Communications in Medicine" (DICOM) standard, for example according to the DICOM PS3.1 2020c standard (or any later or earlier version of said standard).

A medical video report may be configured to illustrate the circumstances, facts and/or findings associated with the medical image study to a human recipient in a video format. In particular, the human recipient may be the patient of the medical image study. Further, the human recipient may be a physician involved in the treatment of the patient, in particular, the referring or treating physician.

The medical video report may comprise a plurality of individual images in the form of an image sequence. Individual images of the medical video report may comprise representations of the medical image study which have been rendered based on the image data comprised in the medical image study. In addition, individual images of the medical video report may comprise rendered annotations relating to observations or findings in the medical image study.

Further, individual images of the medical video report may be based on image data not comprised in the medical image study. In particular, individual images or sequences of images may be pre-configured or may have been pre-produced.

The medical video report may comprise a plurality of (logical) blocks which are each configured to illustrate given facts or circumstances related to the medical image study. For instance, one block may illustrate the reason and circumstances of the underlying examination while other blocks are respectively devoted to illustrating certain findings derived from the examination.

The context information may comprise or characterize circumstances of the case relevant for the generation of the medical video report. In particular, the context information may comprise values for a plurality of pre-defined parameters (as information). The pre-defined parameters may be relevant for the generation of the medical video report.

For instance, the context information may comprise information (e.g., as values for a plurality of pre-defined parameters) characterizing the medical image study. Additionally or alternatively, the context information may comprise information (e.g., as values for a plurality of pre-defined parameters) characterizing an analysis result of the medical image study. Additionally or alternatively, the context information may comprise information (e.g., as values for a plurality of pre-defined parameters) characterizing the human recipient of the medical video report (e.g., in the sense of an expert level of the recipient).

According to some examples, the context information may comprise semantic information extracted from the medical image study, for instance, corresponding to the type of image study, the imaging parameters, the image data shown, the patient, and so forth.

The context information may be automatically extracted from the medical image study (and any supplementary information). Specifically, the values corresponding to one or more pre-defined parameters may be extracted from the medical image study (and any supplementary information).

For instance, the DICOM data structure of the medical image study may be queried for values indicating the type of the image study and/or the body part or organ imaged in the image study and so forth. Similarly, any supplementary information may be mined for relevant parameters such as the age or gender of the patient.

According to some examples, a video script may be conceived as a set of computer-readable instructions which, when executed by a video rendering engine or the like, lead to the generation of a video. Video scripts may define certain image processing operations with which the raw image data of a medical image study may be converted into a video for a human recipient. Further, video scripts may be configured to provide a voice-over of natural language text accompanying the images of the video.

Specifically, video scripts may contain instructions for processing raw image data in order to produce a pre-scripted scene or sequence of individual images which may form a portion of the medical video report. This may include preprocessing parameters (such as contrast enhancement), image rendering parameters (such as volumetric rendering parameters), parameters defining panning, zooming, or cropping of the image data, parameters defining annotations to be added etc. of the images to be rendered for the medical video report.

Each template video script may define a specific type of medical video report to be generated for a specific use-case. Thereby, the use-case may be characterized by the context information. The use-case may relate to the clinical question or decision underlying the medical image study. Further, the use-case may relate to the technical circumstances under which the medical image study was acquired (e.g., modality and imaging parameters) and/or under which the image data shall be transformed into a medical video report.

Each candidate template video script may be preconfigured for a certain use-case. This may be done by an expert user or by an automated process configured to derive a script from existing (verified) medical video reports.

Each one of the candidate template video scripts may be linked to a specific use-case which may be defined by a one or more predefined parameters or by values corresponding to one or more predefined parameters. According to some examples, the pre-defined parameters may be the same pre-defined parameters as in the context information.

According to some examples, the step of selecting an appropriate template video script from the candidate template video scripts may comprise comparing the context information with the candidate template video scripts and matching the candidate template video script to the context information which fits (best) to the context information. According to some examples this may involve comparing values characterizing the context information to corresponding values characterizing the use-case of respective candidate template video script. Specifically, this may involve comparing the values of a set of pre-defined parameters of the context information with corresponding values of the set of pre-defined parameters of the candidate template video script.

It should be noted that the usage of pre-defined parameters is just an example. According to other examples, the right template video script may also be selected based on a semantic matching of the context information with the candidate template video scripts. Specifically, this may comprise obtaining (semantic) information from the candidate template video scripts and selecting the selected template video script based on the obtained (semantic) information, e.g., by comparing the obtained (semantic) information to the context information or corresponding semantic information obtained from/comprised in the context information. The semantic information obtained for the candidate template video scripts may correspond to the context information.

According to some examples, the step of executing the selected template video script may comprise inputting the selected template video script and the medical image study into a rendering engine configured to process image data according to template video scripts. In other words, the step may comprise controlling a rendering engine based on the selected template video script.

According to some examples, providing the medical video report may comprise making it accessible for the human recipient, e.g., by forwarding it to the human recipient or by providing a download link to the human recipient. Further, providing the medical video report may comprise storing the medical video report in an appropriate storage from which it can be accessed by the human recipient, e.g., by download it.

Due to the provision of a plurality of pre-configured template video scripts and the automated selection of an appropriate script based on the patient case, instructions for automatically generating a medical video report can be provided in an efficient way. With that, a medical video report can be provided without requiring a physician to specify instructions for generating a medical video report. Accordingly, the workload of the physician is not significantly increased. What is more, the proposed method seamlessly integrates in the usual reading and reporting workflow, as the medical video report can be automatically generated in parallel to the usual reading and reporting. On the other hand a report in a video format provides the recipient with an instructive summary of the circumstances of the case.

According to some examples, the context information comprises at least one of: a medical reason for the medical image study, a suspected diagnosis for the patient, a medical imaging modality used for the patient, a body part and/or organ depicted in the medical image study, a demographic information of the patient, an expertise level of the human recipient and/or an input of a user based on an interaction of the user with the medical image study or a representation thereof .

According to some examples, the (semantic) information respectively obtained for the candidate template video scripts reflects (corresponds to) the context information and may likewise indicate at least one of: a medical reason for the medical image study, a suspected diagnosis for the patient, a medical imaging modality used for the patient, a body part and/or organ depicted in the medical image study, a demographic information of the patient and/or a expertise level of the human recipient, the respective candidate video template structure was conceived for.

With the above information, a reliable association of appropriate template video scripts can be made for the respective case.

According to some examples, the step of determining the context information comprises obtaining associate data associated to the medical image study and determining the context information additionally based on the associate data.

The associate data may be obtained by querying one or more databases, e.g., based on a digital patient identifier. The one or more databases may be comprised in a healthcare information system such as Picture Archiving or Communication System (PACS), an Electronic Heath Record (EHR) a Radiology Information System (RIS) and/or a Laboratory Information System (LIS) .

The associate data may comprise at least one of: a (prior) medical report of the patient, a prior medical video report, a further (prior) medical image study of the patient, one or more laboratory values of the patient, and/or the electronic health record of the patient.

With the associate data, the context information can be determined more exactly. In turn, the selection of an appropriate template video script can be facilitated.

According to some examples, the medical video report comprises a portion illustrating the one or more aspects of the medical image study to the human recipient, such as a type of, a reason for, a conclusion derived from, and/or a recommendation ensuing from the medical image study.

With that, the human recipient can get more clarity on the circumstances of the medical image study which improves the transparency and the patient engagement.

According to an aspect, the method further comprises obtaining a medical text report relating to the medical image study, wherein, in the step of determining, the context information is extracted from on the medical text report.

Medical text reports may comprise structured or unstructured text relating to medical observations and conclusions for a patient based on an examination result of the patient such as a medical image study or a lab report. Further, medical text reports may also comprise one or more images and/or graphs extracted from an examination result.

The medical text report may be an already exiting and finalized report which may be actively pulled from a database (e.g., as associate data). According to other examples, the medical text report may be in the process of being generated "on the fly" by a user. Thereby, the user may be someone currently reviewing the medical image study, such as a physician, in particular, a radiologist. Thereby, the user is generally different than the human recipient. User interactions for generating the medical text report may be logged and used for determining the context information.

If not indicated otherwise, the term "medical report" refers to a textual medical report or medical text report and not to medical video reports.

Medical reports may provide a concise and well accessible representation of the circumstances of the case. As such, medical reports provide a good basis for extracting the context information. In turn, this enables to efficiently find suited template video scripts.

According to some examples, the medical text report is based on a text report template, wherein the context information comprises the text report template (or the type of the text report template).

In other words, the template video script is selected according to the text report template used for the medical text report. This has the advantage that a medical video report may be generated which optimally complements the medical text report.

According to some examples, the text report template is selected from a plurality of pre-configured text report templates, wherein the method comprises providing the plurality candidate template video scripts so that each candidate template video script is associated to one of the plurality of pre-configured text report templates, and, in the step of selecting the template video script, the template video script associated to the text report template is selected from the candidate template video scripts.

In other words, the candidate template video scripts held available match the report templates which are to be used for generating a medical text report. This renders the selection process of the template video script unambiguous. Further, it can be ensured that the medical video report optimally complements the medical text report.

According to an aspect, the context information comprises a medical finding of a predefined finding type, and the selected template video script encodes instructions for image processing steps of a medical image study in order to generate a video visualizing a medical finding of the predefined finding type for the human recipient.

According to some examples, in the step of selecting, the selected template video script is selected based on the predefined fining type. Thereby, according to some examples, each of the candidate template video scripts may encode instructions for visualizing different predefined finding types, wherein, in the step of selecting, at least one template video script is identified form the candidate template video scripts which encodes instructions for visualizing the predefined finding type.

According to an aspect, a computer-implemented method for providing a medical video report is provided, which comprises the steps of: receiving a medical image study of a patient, obtaining a medical finding pertaining to the image study, selecting, based on the finding type, a template video script from a plurality of candidate template video scripts, each candidate template video script encoding instructions for image processing steps of a medical image study in order to generate a video visualizing a medical finding of a specific finding type for a human recipient, applying the selected video template element on the medical image study so as to generate the medical video report, and providing the medical video report.

Each medical finding may relate to corresponding image data in the medical image study. A medical finding may indicate a certain condition or pathology of the patient. The condition or pathology may be relevant for the diagnosis of the patient.

A medical finding may relate to an anatomical structure that differentiates the patient from other patients. Medical findings may be located within different organs of the patient (e.g., within the lung of a patient, or within the liver of a patient) or in between the organs of the patient. In particular, a medical finding may also relate to a foreign body.

In particular, a medical finding may relate to a neoplasm (also denoted as "tumor"), in particular, a benign neoplasm, an in-situ neoplasm, a malignant neoplasm and/or a neoplasm of uncertain/unknown behavior. In particular, a medical finding may relate to a nodule, in particular, a lung nodule. In particular, a medical finding may relate to a lesion, in particular, a lung lesion.

Medical findings may be obtained from an existing medical text report, or a medical text report currently being created by a user. Further medical findings may be obtained by applying an image processing algorithm (i.e., a computer aided detection function) configured to identify medical findings in medical image studies. Further, medical findings may be directly obtained from the user interacting with the medical image study (e.g., by applying measurement tools to a representation/visualization of the medical image study).

Medical findings may be classified according to their type or category. This type or category is called "finding type". A finding type may specify the general nature of the medical finding. Further, the finding type may specify the anatomy or organ in which a medical finding has been found. According to some implementations, the finding type may also be conceived as a label of the medical finding. For instance, finding types may be lung nodule, liver nodule, cyst, rib fracture, undefined lesion, and so forth.

By relying on finding types (and preconfiguring template video scripts accord a plurality of finding types) template video scripts may be efficiently provided and selected. With that, appropriate video sequences can be produced which tightly reflect the circumstances of the case.

According to some examples, the image processing steps comprise: determine an organ corresponding to the medical finding, segment the organ in the medical image study by applying an image segmentation algorithm so as to generate segmented image data, render a predefined sequence of images based on the segmented image data wherein the medical finding is highlighted in at least part of the images of the sequence.

With that, medical findings can be appropriately carved out and brought to the attention of the human recipient.

According to some examples, the context information further comprises supplementary information related to the medical finding and the selected video template is further configured to translate the supplementary information into the video visualizing the medical finding, so that the supplementary information is perceivable for the human recipient, preferably in the form of a visual marking. According to some examples, the supplementary information comprises at least one of: a criticality of the medical finding, a progression of the medical finding over time, a classification of the medical finding, and/or a medical scoring of the medical finding.

According to some examples, the selected template video script may additionally encode instructions for image processing steps of a medical image study in order to generate a video visualizing a further medical finding of a finding type different from the predefined finding type.

According to some examples, the further finding may relate to an incidental finding. With that, further or incidental findings may be visualized even though the template video script may primarily relate to another finding or another topic such as a therapy video explaining a required therapy to the human recipient.

According to some examples, the context information comprises a user input relating to the medical image study. Specifically, the user input may be input via a user interface and may be directed to a medical finding visible in a representation of the medical image study visualized in the user interface.

The representation may be a two-dimensional representation image rendered from the medical image study for displaying to a user in a user interface. The representation may comprise a plurality of image pixels. In particular, the representation may be a two-dimensional rendering of the medical image. Two-dimensional renderings may, in general, rely on known rendering procedures, such as ray-casting, ray-tracing, texture-rendering or the like. According to some examples, the rendering may be such that already identified medical findings and/or any candidate medical findings are displayed in conjunction with the image data of the medical image.

The user input may be any input directed to designate a medical finding. The user input may comprise a voice command or any other, in particular, physical input into a user interface, in particular, a graphical user interface. For instance, the user may use input devices like a computer-mouse, a trackball device, a smart pen, a touch pad, a touch sensitive display, etc. Further, the user input may be captured by eye tracking or by tracking gestures. The user input may, in particular, comprise designating a medical finding directly in the representation, e.g., by clicking, drawing contours, or invoking a measurement tool in a specific location in the representation. The user input may be such that it fully lines out the medical finding or such that it indicates only parts or even only a point in the representation which is then automatically related to a possible medical finding.

According to some implementations the user input may comprise a plurality of individual user interactions with a user interface (such as user inputs with regard to the representation, displaying settings, general settings, measurements, etc.).

According to some examples, the candidate template video scripts respectively comprise a plurality of optionally activable video template structures each encoding instructions for providing a portion of a medical video report based on a medical image study, the step of executing comprises activating, based on the context information, at least one of the video template structures of the selected video script, and the medical video report is generated based on the at least one activated video template structure.

In other words, each template video script may comprise a plurality of independent blocks or elements which are in principle suited to visualize individual facets of a use-case described by the context information. Dependent on the actually available information, these blocks may be selectively activated. Then, each activated block may correspond to a portion of the final medical video report.

According to some examples, individual structures of a script may relate to different medical findings and/or standard video sequences for a certain use-case. For instance, a script for an X-Ray scan of the lung of a patient may comprise structures relating to general explanations regarding X-Ray examinations and structures for possible lung-related findings such as lung nodules or interstitial lung diseases.

Thereby, individual template structures may be shared between different template video scripts. For instance, each script relating to a CT exam as medical image study may comprise the same template structure with some general explanations regarding CT exams.

By pre-configuring different structures in the scripts which can be activated depending on the respective use-case, the template video scripts become more flexible. Accordingly, they integrate very well into existing workflows.

According to an aspect, the optionally activable video template structures of the candidate template video scripts comprise finding-specific video template structures with instructions for visualizing a medical finding of a predefined finding type. The method further comprises obtaining at least a medical finding of a given finding type pertaining to the medical image study from the medical image study and/or any associate information of the medical image study, and the step of activating comprises activating, based on the given finding type, at least one finding-specific video template structure of the selected template video script the predefined finding type of which matches the given finding type.

By pre-configuring different instructions for different finding types, the template video script is rendered more flexible and smoothly integrates into existing workflows.

According to some examples, the step of activating at least one a video template structure comprises determining a property of the medical finding and activating at least one video template structure based on the property. According to some examples, the property may comprise at least one of: a size of the medical finding, a criticality of the medical finding, progression of the medical finding over time, a classification of the medical finding, and/or a medical scoring of the medical finding. With that, building blocks of the medical video report may be selected which reflect the properties of the medical finding. For instance, this allows to generate different video sequences if a nodule is smaller than a certain size as compared to a case where the nodule is larger.

According to an aspect, the step of obtaining the at least one medical finding comprises, querying if the medical image study indicates one or more medical findings of the predefined finding types.

In other words, the medical image study may be actively queried for possible findings based on the selected template video script. With that, another safety layer can be provided making sure that no relevant findings of a use-case are overlooked.

According to some examples, the step of querying comprises automatically applying, to the medical image study, an image processing algorithm configured to identify at least one of the predefined medical findings the selected template video script comprises video template structures for.

Automatically invoking a computer aided detection tool (in the form of the image processing algorithm) further reduces the workload for the user and the reporting workflow can be further automated.

According to some examples, the step of querying comprises notifying the user of at least one of the predefined medical findings the selected template video script comprises video template structures for.

Based on the notification, the user may then look if the medical image study comprises any indications for the at least one finding. With that, the user is provided with additional guidance what to look for.

According to some examples, the step of querying comprises processing a medical text report related to the medical image study so as to determine if the medical text report indicates one or more medical findings of the predefined finding types.

According to an aspect, the candidate template video scripts respectively comprise at least a video template structure relating to a preexisting video sequence, and the step of executing comprises retrieving the preexisting video sequence from a repository configured to store a plurality of preexisting video sequences, and including the preexisting video sequence in the medical video report.

According to some examples, the method may further comprise providing the repository.

With the preexisting video sequences, content can be held available which relates to standard video snippets which are independent of the image data in the medical image study. This may relate to explanatory or introductory sequences explaining a certain image examination. Accordingly, content may be pre-produced and reused which renders the method more efficient and consumes fewer computing resources.

According to an aspect, the method further comprises obtaining natural language text from the context information and/or the medical image study, generating a voice-over based on the natural language text, and including the voice-over in the medical video report.

According to some examples, obtaining natural language text may comprise (directly) extracting the natural language text from structured or unstructured text elements comprised in the medical image study or the context information. Such text elements may by DICOM tags of the medical image study or text fields of a medical text report. According to other examples, obtaining natural language text may comprise transferring non-text data in natural language text. According to some examples, obtaining may comprise applying a text generation function to the medical image study and/or the context information so as to obtain natural language text.

According to some examples, the step of generating the voice-over may comprise inputting the natural language text in a chat function configured to generate speech output based on natural language text. Thereby, the chat function may be configured to filter, summarize, or re-word the input natural language text so as to generate an output suited for an accompanying voice-over for the medical video report. Further, the step of generating the voice-over may comprise generating a soundtrack of the video medical report based on the voice-over.

According to some examples, the text generation function and/or the chat function may comprise a transformer network and, in particular, a large language model such as BERT or ChatGPT.

A transformer network is a neural network architecture generally comprising an encoder, a decoder or both an encoder and decoder. In some instances, the encoders and/or decoders are composed of several corresponding encoding layers and decoding layers, respectively. Within each encoding and decoding layer is an attention mechanism. The attention mechanism, sometimes called self-attention, relates data items (such as words or pixels) within a series of data items to other data items within the series. The self-attention mechanism for instance allows the model to examine a group of voxels or word within a medical image or sentence and determine the relative importance other groups of voxels or words within that medical image or sentence have to the group of voxels or word being examined.

The encoder, in particular, may be configured to transform the input (a medical image or text) into a numerical representation. The numerical representation may comprise a vector per input token (e.g., per word). The encoder may be configured to implement an attention mechanism so that each vector of a token is affected by the other tokens in the input. In particular, the encoder may be configured such that the representations resolve the desired output of the transformer network.

The decoder, in particular, may be configured to transform an input into a sequence of output tokens. In particular, the decoder may be configured to implement a masked self-attention mechanism so that each vector of a token is affected only by the other tokens to one side of a sequence. Further, the decoder may be auto-regressive meaning in that intermediate results (such as a previously predicted sequence of tokens) are fed back.

According to some examples, the output of the encoder is input into the decoder.

Further, the transformer network may comprise a classification module or unit configured to map the output of the encoder or decoder to a set of learned outputs such as the natural language text or the voice-over text.

Training of a transformer model according to some examples may happen in two stages, a pretraining and a fine-tuning stage. In the pretraining stage, a transformer model may be trained on a large corpus of data to learn the underlying semantics of the problem. Such pre-trained transformer models are available for different languages. For certain applications described herein, the fine-tuning may comprise further training the transformer network with medical texts with expert annotated meanings and/or medical ontologies such as RADLEX and/or SNOMED. With the latter, in particular, the transformer model according to some examples may learn typical relations and synonyms of medical expressions.

For a review on transformer networks, reference is made to Vaswani et al., "Attention Is All You Need", in arXiv: 1706.03762, June 12, 2017, the contents of which are herein included by reference in their entirety.

By providing the voice-over, the medical video report can be made more instructive and better accessible for the human recipient. At the same time, due to the automated generation of the voice-over, the workload of the clinical personnel is not increased.

An advantage of transformer networks is that, due to the attention mechanism, transformer networks can efficiently deal with long-range dependencies in input data. Further, encoders used in transformer networks are capable of processing data in parallel which saves computing resources in inference. Moreover, decoders of transformer networks, due the auto-regression, are able to iteratively generate a sequence of output tokens with great confidence.

According to some examples, the step of generating the voice-over may comprise providing a voice-over template and populating the voice-over template based on the natural language text. According to some examples, providing the voice-over template may further comprise providing a voice-over template for each video template structure, and selecting at least one voice-over template according to the activated video template structure.

According to an aspect, in the step of obtaining natural language text, the natural language text is extracted from the medical text report.

By mining a medical text report, information immediately relevant for the medical image study can be gathered, which, in addition, does not require much processing for turning it into a voice-over. Specifically, by relying on a medical text report, passages of the report may be verbatim adopted into the voice-over.

According to some examples, the step of generating the voice-over may comprise listening to a speech input by a user into a user interface and generating the voice-over based on the speech input.

In other words, the voice-over may be at least partially generated from direct dictation of a user, which simplifies the workflow. Since physicians anyway tend to dictate their findings for generating a medical text report, this furthermore does not impact on the workload of the user.

According to an aspect, the step of obtaining natural language text comprises providing a mapping function configured to map medical finding onto a structured text, and generating the natural language text by applying the mapping function to the medical finding.

According to some examples, the mapping function may be the text generation function as herein described. In particular, the mapping function may comprise a transformer network.

The usage of a mapping function enables the structured processing of the available information into actionable results which may be readily used by the ensuing video generation.

According to an aspect, the step of generating the voice-over comprises providing a chat function configured to transcribe natural language text from a first version to a second version, applying the chat function to the natural language text so as to transcribe the natural language text into the second version, and, in the step of generating the voice-over, the voice-over is generated based on the natural language text of the second version.

The chat function may be configured according to the aspects and examples for chat functions herein described. In particular, the chat function may comprise a transformer network and/or a large language model.

According to some examples, the first version of the natural language text may be the text as extracted from the available data. According to some examples, the second version may be adapted to the human recipient. According to some examples, the second version may be adapted to the human recipient based on information on the human recipient comprised in the context information. According to some examples, the second version may correspond to a different language, a different complexity level, in particular, a simpler language, and/or a different level of text summarization, in particular, a more concise language, than the first version. The simpler language may be characterized by an avoidance of technical terms comprised in the first version and/or their replacement by layperson terms in the second version.

According to some examples, the second version, and, in particular, at least one target property of the second version may be determined based on the context information and/or the selected template video script and/or the activated video template structures (e.g., by the appropriately configured chat function). The at least one target property may comprise a target complexity level of the language used, a target length of the resulting natural language text in the second version, a target language of the natural language text in the second version and so forth.

By transferring the available text information before generating the audio track of the medical video report on that basis, the voice-over can be automatically adapted to the needs of, e.g., the human recipient. With that, a readily usable result is generated without requiring a user to interfere.

According to an aspect, the medical image study comprises a three-dimensional medical image data set, the template video script encodes instructions for volumetric image rendering, in particular, implementing a path-tracing- or ray-casting-based rendering process, and the medical video report comprises one or more images generated via volumetric image rendering.

In ray casting, simulated rays emanating from the eye of an imaginary observer are transmitted through the examined body or the examined object (cf. Levoy: "Display of Surfaces from Volume Data", IEEE Computer Graphics and Applications, issue 8, no. 3, May 1988, pages 29-37). Along the rays, RGBA values are determined for sampling points from the voxels and combined to form pixels for a two-dimensional image by means of alpha compositing or alpha blending. Here, the letters R, G and B in the expression RGBA represent the color components red, green, and blue, from which the color contribution of the corresponding sampling point is composed. A represents the ALPHA value, which represents a measure for the transparency at the sampling point. The respective transparency is used in the superposition of RGB values at sampling points to form the pixel. Lighting effects are usually considered by means of a lighting model within the scope of a method referred to as "shading".

A further method for volume rendering is the so-called path tracing method (cf. Kajiya: "The rendering equation", ACM SIGGRAPH Computer Graphics, issue 20, no. 4, August 1986, pages 143-150). Here, a plurality of simulated rays is shot into the volume data per visualization pixel, said simulated rays then interacting with the volume, i.e., are reflected, refracted, or absorbed, wherein at least one random ray is generated every time (except in the case of absorption). Each simulated ray thus finds its path through the volume data. The more virtual rays are used per visualization pixel, the better the image. Here, use can be made, in particular, of the processes and methods described in EP 3 178 068 B1. The content of EP 3 178 068 B1 is incorporated herein in full by reference.

Accordingly, the template video script may specify parameters for the path - and/or ray-casting process such as zoom levels, viewing angles, transfer functions, texture values, number of rays, transparency levels, scene illuminations and so forth.

On the one hand, such methods allow particularly realistic visualizations to be generated. This provides the human recipient with an instructive picture of the imaging examination and its outcome. On the other hand, since the volumetric image rendering is triggered automatically, the user does not need to get involved which spares the user of familiarizing herself or himself with the subtleties of a volumetric rendering pipeline (which may be complex).

According to some examples, the step of providing the medical video report may comprise providing the medical video report to a user for review in a user interface, receiving feedback from the user indicating a consent to the medical video report, and releasing the medical video report to the human recipient based on the feedback. According to some examples, providing the medical video report to the user comprises providing the voice-over as text for review.

According to some examples, the step of providing the medical video report to the user may comprise visualizing different blocks of the medical video report (which blocks respectively may relate to an individual video template structure), according to some examples, by thumbnails. According to some examples, the feedback includes a consent for individual blocks, and releasing comprises including those blocks in the medical video report for which a consent is included in the feedback.

By showing the video medical report to a user before releasing it to the human recipient, the end control can be performed by a user. With that, it can be ensured that the medical video report has a sufficient quality and complies with the applicable regulations. At the same time, the end control is enabled at a manageable workload. In particular, this is the case if different blocks of the medical video report are visually presented as this allows for a good overview of the contents "at a glance".

According to an aspect, a system for providing a medical video report is provided. The system comprises an interface unit and a computing unit. The interface unit is configured to receive a medical image study of a patient and to provide the medical video report. The computing unit is configured to determine, based on the medical image study, a context information pertaining to the medical image study. Further, the computing unit is configured to select, based on the context information, a template video script from a plurality of candidate template video scripts, each candidate template video script encoding instructions for image processing steps of a medical image study in order to generate a video visualizing a medical diagnosis to a human recipient. Further, the computing unit is configured to execute the selected template video script on the medical image study so as to generate the medical video report. Further, the computation unit is configured to provide the medical video report.

The computing unit may be realized as a data processing system or as a part of a data processing system. Such a data processing system can, for example, comprise a cloud-computing system, a computer network, a computer, a tablet computer, a smartphone and/or the like. The computing unit can comprise hardware and/or software. The hardware can comprise, for example, one or more processors, one or more memories, and combinations thereof. The one or more memories may store instructions for carrying out the method steps according to the invention. The hardware can be configurable by the software and/or be operable by the software. Generally, all units, sub-units or modules may at least temporarily be in data exchange with each other, e.g., via a network connection or respective interfaces. Consequently, individual units may be located apart from each other. Further, the computing unit may be configured as an edge device.

According to some examples, the computing unit may comprise a context extraction module or unit configured to extract the context information from the medical image study and/or any associate data. Further, the computing unit may comprise a video content module or unit configured to select a template video script according to the context information. Further, the computing unit may comprise a rendering module or unit configured to render the medical video report based on the selected script and the medical image study. According to some examples, the computing unit may comprise a voice-over module or unit configured to generate text based on the available information and data, that is, the context information, the medical image study, and any associate information (such as medical findings identified in the medical image study). According to some examples, the voice-over module may comprise a findings2text engine configured to extract natural language text from the available information and a chat engine configured to transcribe the extracted text into a version suited for the soundtrack of the medical video report.

The interface unit may comprise an interface for data exchange with a template database for parsing the template database and retrieving template video scripts. The interface unit may be further adapted to interface with one or more users of the system, e.g., by receiving the query and/or displaying the result of the processing, i.e., the information, to the user (e.g., in a graphical user interface).

The systems may be adapted to implement the inventive method in their various aspects for modifying medical image data. The advantages described in connection with the method aspects and examples may also be realized by the correspondingly configured systems' components. Accordingly, the advantages described in connection with the method-aspects and examples are also attributable to the corresponding systems.

According to another aspect, the present invention is directed to a computer program product comprising program elements which induce a computing unit of a system configured to provide a medical video report to perform the steps according to one or more of the above method aspects and examples, when the program elements are loaded into a memory of the computing unit.

According to another aspect, the present invention is directed to a computer-readable medium on which program elements are stored that are readable and executable by a computing unit of a system configured to provide a medical video report according to one or more method aspects and examples, when the program elements are executed by the computing unit.

The realization of the invention by a computer program product and/or a computer-readable medium has the advantage that already existing providing systems can be easily adapted by software updates in order to work as proposed by the invention.

The computer program product can be, for example, a computer program or comprise another element next to the computer program as such. This other element can be hardware, e.g., a memory device, on which the computer program is stored, a hardware key for using the computer program and the like, and/or software, e.g., a documentation or a software key for using the computer program. The computer program product may further comprise development material, a runtime system and/or databases or libraries. The computer program product may be distributed among several computer instances.

Characteristics, features, and advantages of the above-described invention, as well as the manner they are achieved, become clearer and more understandable in the light of the following description of embodiments, which will be described in detail with respect to the figures. This following description does not limit the invention on the contained embodiments. Same components, parts or steps can be labeled with the same reference signs in different figures. In general, the figures are not drawn to scale. In the following:
- FIG. 1: schematically depicts a system for providing a medical video report according to an embodiment,
- FIG. 2: schematically depicts a method for providing a medical video report according to an embodiment,
- FIG. 3: schematically depicts a data flow diagram in a method for providing a medical video report according to an embodiment,
- FIG. 4: schematically depicts a template video script according to an embodiment,
- FIG. 5: schematically depicts optional method steps in a method for providing a medical video report according to an embodiment,
- FIG. 6: schematically depicts optional method steps in a method for providing a medical video report according to an embodiment,
- FIG. 7: schematically depicts a data flow diagram in a method for providing a medical video report according to an embodiment, and
- FIG. 8: schematically depicts a data processing function according to an embodiment.

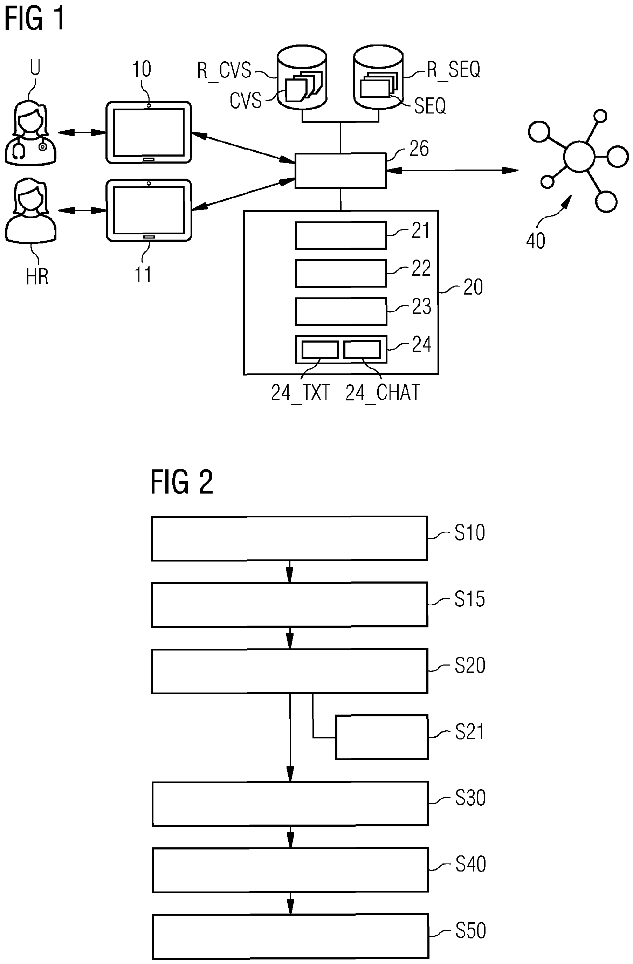

Figure 1 depicts a system 1 for providing a medical video report MVR for a human recipient HR. In this regard, system 1 is adapted to perform the methods according to one or more embodiments, e.g., as further described with reference to Figures 2 to 8.

A user U of system 1 (in the sense of an operator controlling the system 1), according to some examples, may generally relate to a healthcare professional such as a physician, clinician, technician, radiologist and so forth. A human recipient HR (in the sense of a person designated to receive a processing result of the system 1), according to some examples, may generally relate to a patient or (healthcare) personnel different than the user such as a referring physician.

System 1 comprises a user interface 10 (as part of the interface unit), a recipient interface 11 (as part of the interface unit), and a processing system 20 (as part of the computing unit). Further, system 1 may comprise or be connected to a medical information system 40. The medical information system 40 may generally be configured for acquiring and/or storing and/or forwarding medical image studies MIS and supplementary (non-image) information. For instance, medical information system 40 may comprise one or more archive/review station (not shown) for medical image studies MIS. The archive/review stations may be embodied by one or more databases. In particular, the archive/review stations may be realized in the form of one or more cloud storage modules. Alternatively, the archive/review stations may be realized as a local or spread storage, e.g., as a PACS (Picture Archiving and Communication System). According to some examples, medical information system 40 may also comprise one or more medical imaging modalities (not shown), such as a computed tomography system, a magnetic resonance system, an angiography (or C-arm X-ray) system, a positron-emission tomography system, a mammography system, an X-ray system, or the like.

Medical image studies MIS may be three-dimensional image data sets acquired, for instance, using an X-ray system, a computed tomography system or a magnetic resonance imaging system or other systems. The image information may be encoded in a three-dimensional array of m times n times p voxels. Medical image studies MIS may include a plurality of image slices which are stacked in a stacking direction to span the image volume covered by the medical image study MIS.

Further, medical image studies MIS may comprise two-dimensional medical image data with the image information being encoded in an array of m times n pixels. According to some examples, these two-dimensional medical images may have been extracted from three-dimensional medical image studies MIS.

An ensemble of voxels or pixels may be designated as image data of the respective medical image study in the following. In general, any kind of imaging modalities and scanners may be used for acquiring such image data. Generally, medical image studies MIS may show a body part or an anatomical region or an anatomic object of a patient which may comprise various anatomies and organs. Considering the chest area as a body part, medical image studies MIS might, for instance, depict the lung lobes, the rib cage, the heart, lymph nodes, and so forth.

Medical image studies MIS may be formatted according to the DICOM format. DICOM (=Digital Imaging and Communications in Medicine) is an open standard for the communication and management of medical imaging information and related data in healthcare informatics. DICOM may be used for storing and transmitting medical images and associated information enabling the integration of medical imaging devices such as scanners, servers, workstations, printers, network hardware, and picture archiving and communication systems (PACS). It is widely adopted by clinical syndicates, hospitals, as well as for smaller applications like doctors' offices or practices. A DICOM data object consists of a number of attributes, including items such as the patient's name, ID, etc., and also special attributes containing the image pixel data and metadata extracted from the image data.

Supplementary information (or associate data) may be any data providing additional information relating to the patient and/or the medical image study MIS. The supplementary information may comprise image data such as other medical image studies MIS of the patient which were, for instance, acquired at an earlier point in time than the medical image study MIS under consideration. Further the supplementary information may comprise non-image data or data with mixed-type contents comprising medical images and non-image contents such as text. Non-image data may relate to non-image examination results such as lab data, vital signs records (comprising, e.g., ECG data, blood pressure values, ventilation parameters, oxygen saturation levels) and so forth. Moreover, the supplementary information may comprise structured and unstructured medical text reports MTR relating to prior examinations or the current examination of the patient. Further, non-image data may comprise personal information of the patient such as gender, age, weight, insurance details, and so forth.

The supplementary information may be available in the form of one or more electronic medical reports of the patient. The supplementary information may be stored in the healthcare information system 40. For instance, the supplementary information may be stored in dedicated databases of the healthcare information system 40 such as laboratory information system (LIS) or an electronic health/medical record database.

User interface 10 may comprise a display unit and an input unit. User interface 10 may be embodied by a mobile device such as a smartphone or tablet computer. Further, user interface 10 may be embodied as a workstation in the form of a desktop PC or laptop. The input unit may be integrated in the display unit, e.g., in the form of a touch screen. As an alternative or in addition to that, the input unit may comprise a keyboard, a mouse or a digital pen and any combination thereof. The display unit may be configured for displaying a representation of the medical image study MIS, for displaying and editing a medical text report MTR, for playing a medical video report MVR, for displaying thumbnails of the medical video report MVR, and for receiving any user input, e.g., for approving a medical video report MVR or parts of it.

User interface 10 may further comprise an interface computing unit configured to execute at least one software component for serving the display unit and the input unit in order to provide a graphical user interface for allowing the user to select a target patient's case to be reviewed and making various inputs. In addition, the interface computing unit may be configured to communicate with medical information system 40 or processing system 20 for receiving the medical image studies MIS and any supplementary information. The user U may activate the software component via user interface 10 and may acquire the software component, e.g., by downloading it from an internet application store. According to an example, the software component may also be a client-server computer program in the form of a web application running in a web browser. The interface computing unit may be a general processor, central processing unit, control processor, graphics processing unit, digital signal processor, three-dimensional rendering processor, image processor, application specific integrated circuit, field programmable gate array, digital circuit, analog circuit, combinations thereof, or other now known devices for processing image data. User interface 10 may also be embodied as a client.

Recipient interface 11 may comprise a display unit and an input unit. Recipient interface 11 may be embodied by a mobile device such as a smartphone or tablet computer. Further, recipient interface 11 may be embodied as a workstation in the form of a desktop PC or laptop. The input unit may be integrated in the display unit, e.g., in the form of a touch screen. As an alternative or in addition to that, the input unit may comprise a keyboard, a mouse or a digital pen and any combination thereof. The display unit may be configured for playing a medical video report MVR.

Recipient interface 11 may further comprise an interface computing unit configured to execute at least one software component for serving the display unit and the input unit in order to provide a graphical user interface for allowing the user to receive and play a medical video report MVR. The user may activate the software component via recipient interface 11 and may acquire the software component, e.g., by downloading it from an internet application store. According to an example, the software component may also be a client-server computer program in the form of a web application running in a web browser. The interface computing unit may be a general processor, central processing unit, control processor, graphics processing unit, digital signal processor, three-dimensional rendering processor, image processor, application specific integrated circuit, field programmable gate array, digital circuit, analog circuit, combinations thereof, or other now known devices for processing image data. Recipient interface 11 may also be embodied as a client.

Processing system 20 may comprise sub-units 21-24 configured to process the medical image studies MIS and supplementary information in order to provide a medical video report MVR.

Processing system 20 may be a processor. The processor may be a general processor, central processing unit, control processor, graphics processing unit, digital signal processor, three-dimensional rendering processor, image processor, application specific integrated circuit, field programmable gate array, digital circuit, analog circuit, combinations thereof, or other now known device for processing image data. The processor may be single device or multiple devices operating in serial, parallel, or separately. The processor may be a main processor of a computer, such as a laptop or desktop computer, or may be a processor for handling some tasks in a larger system, such as in the medical information system or the server. The processor is configured by instructions, design, hardware, and/or software to perform the steps discussed herein. The processing system 20 may be comprised in the user interface 10. Alternatively, processing system 20 may comprise a real or virtual group of computers like a so called `cluster' or 'cloud'. Such server system may be a central server, e.g., a cloud server, or a local server, e.g., located on a hospital or radiology site. Further, processing system 20 may comprise a memory such as a R_AM for temporally loading the medical image studies MIS. According to some examples, such memory may as well be comprised in user interface 10.

Sub-unit 21 is configured to extract the relevant context information CI from the medical image study MIS and any supplementary information. The context information CI may comprise the "reason for the medical image study" and additional relevant information pertaining to the case such as medical findings identified and/or information pertaining to the human recipient HR. To extract the context information CI, sub-unit 21 may be configured to run an accordingly configured function CI_FU according to some examples.

Sub-unit 22 is configured to select an appropriate template video script TVS from a plurality of candidate template video scripts CVS based on the context information CI. Thereby, the template video scripts TVS encode instructions for rendering a medical video report MVR which fits the respective use-case (as characterized by the context information CI). Further, sub-unit 22 may be configured to activate and deactivate blocks withing a selected template video script TVS based on the available information.

In other words, sub-unit 22 may be configured to select the right template video script TVS and, optionally, edit the same based on the medical findings comprised in the medical image study MIS and any supplementary information. Another expression for sub-unit 22 may be finding-to-video-engine.

To match the context information CI with the appropriate template video script TVS and select individual blocks within a template video script TVS, sub-unit 22 may be configured to run an accordingly configured selection function TVS_FU.

Sub-unit 23 may be conceived as a rendering module or unit. Sub-unit 23 may configured to process instructions comprised in the template video scripts TVS so as to render a medical video report MVR based on the medical image study MIS and any supplementary information. Another expression for sub-unit 23 may be rendering engine. To process the medical image study MIS and the supplementary information according to the selected (and optionally edited) template video script TVS, sub-unit 23 may be configured to run an accordingly configured rendering function REN_FU. According to some examples, the rendering function REN_FU may implement a volume rendering algorithm which may, in particular, be based on path-tracing or ray-casting.

Sub-unit 24 may be conceived as voice-over module or unit. It is configured to generate an audio track for the medical video report MVR comprising a speech output with further explanations for the human recipient HR. In other words, sub-unit 24 may be configured to extract information from the medical image study MIS, the context information CI, and/or any supplementary information and transfer this information into speech suited for accompanying the video frames.

To this end, according to some examples, sub-unit 24 may comprise two sub-modules 24_TXT and 24_CHAT. While sub-module 24_TXT may be conceived as a finding-to-text engine configured to distill medically relevant information from the available data and transfer it into (machine-readable) text TXT, sub-module 24_CHAT may be seen as a chat engine configured to transfer the text into an appropriate speech output VO (adapted to the medical video report MVR and the needs of the human recipient).

According to some examples, sub-module 24_TXT may run a text generation function TXT_FU configured to turn medical findings and other relevant information into structured or unstructured text TXT. According to some examples, sub-module 24_CHAT may run a chat function CHAT_FU configured to transcribe text into a version suited as a basis for a speech output / voice-over VO.

The designation of the distinct sub-units 21-24 is to be construed by way of example and not as a limitation. Accordingly, sub-units 21-24 may be integrated to form one single unit (e.g., in the form of "the computing unit") or can be embodied by computer code segments configured to execute the corresponding method steps running on a processor or the like of processing system 20. The same holds true with respect to the interface computing unit. Each sub-unit 21-24 and the interface computing unit may be individually connected to other sub-units and/or other components of the system 1 where data exchange is needed to perform the method steps.

Processing system 20 and the interface computing unit(s) together may constitute the computing unit of the system 1. Of note, the layout of this computing unit, i.e., the physical distribution of the interface computing unit and sub-units 21-24 is, in principle, arbitrary. Specifically, processing system 20 may also be integrated in user interface 10. As already mentioned, processing system 20 may alternatively be embodied as a server system, e.g., a cloud server, or a local server, e.g., located on a hospital or radiology site. According to such implementation, user interface 10 could be designated as a "frontend" or "client" facing the user, while processing system 20 could then be conceived as a "backend" or server. Communication between user interface 10 and processing system 20 may be conducted using the https-protocol, for instance. The computational power of the system may be distributed between the server and the client (i.e., user interface 10). In a "thin client" system, the majority of the computational capabilities exists at the server. In a "thick client" system, more of the computational capabilities, and possibly data, exist on the client.

Further, system 1 may comprise a template database R_CVS serving as a repository for a plurality of candidate template video scripts CVS. The template database R_CVS is a storage device such a cloud or local storage serving as an archive for template video scripts TVS.

Still further, system 1 may comprise a standard video database R_SEQ serving as a repository for readily rendered standard video sequences SEQ which may be appended to the video material newly rendered based on the medical image study MIS. The standard video database R_SEQ is a storage device such a cloud or local storage serving as an archive for standard video sequences SEQ.

Individual components of system 1 may be at least temporarily connected to each other for data transfer and/or exchange. User interface 10 communicates with processing system 20 via (data) interface 26 to exchange, e.g., medical image studies MIS, the medical text reports MTR, the final medical video report MVR, or any user input made. For example, processing system 20 may be activated on a request-base, wherein the request is sent by user interface 10. Further, recipient interface 11 communicates with processing system 20 via (data) interface 26 to receive the medical video report MVR. Further, processing system 20 may communicate with medical information system 40 in order to retrieve a target patient's case. As an alternative or in addition to that, user interface 10 may communicate with medical information system 40 directly. Medical information system 40 may likewise be activated on a request-base, wherein the request is sent by processing system 20 and/or user interface 10. Data interface 26 for data exchange may be realized as hardware- or software-interface, e.g., a PCI-bus, USB, or fire-wire. Data transfer may be realized using a network connection. The network may be realized as local area network (LAN), e.g., an intranet or a wide area network (WAN). Network connection is preferably wireless, e.g., as wireless LAN (WLAN or Wi-Fi). Further, the network may comprise a combination of different network examples. Interface 26 for data exchange together with the components for interfacing with the user be regarded as constituting an interface unit of system 1.

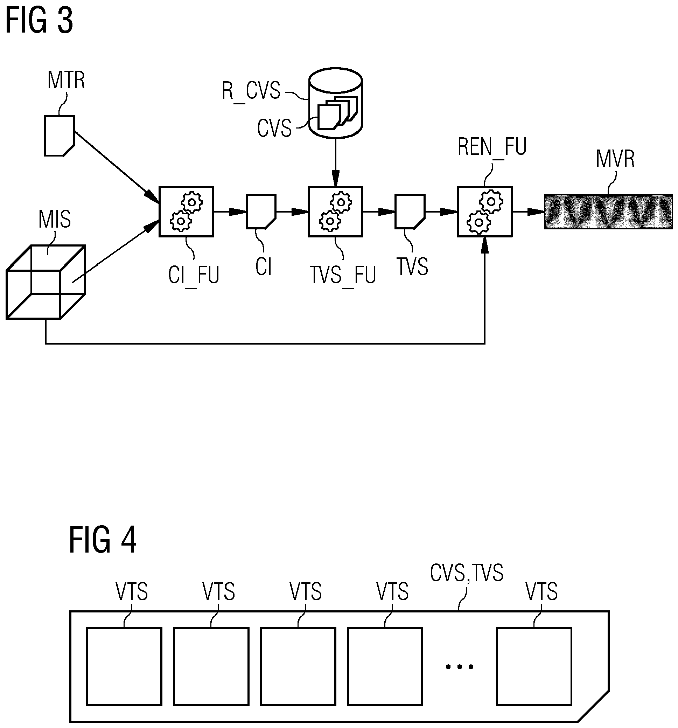

Figure 2 depicts a method for providing a medical video report MVR according to an embodiment. Corresponding data streams are illustrated in Figure 3. The method comprises several steps. The order of the steps does not necessarily correspond to the numbering of the steps but may also vary between different embodiments of the present invention. Further, individual steps or a sequence of steps may be repeated.

In a first step S10, the medical image study MIS is received. This may involve selecting the medical image study MIS from a plurality of cases, e.g., stored in the medical information system 40. The selection may be performed manually by a user U, e.g., by selecting appropriate image data in a graphical user interface running in the user interface 10. Alternatively, the medical image study MIS may be provided to the computing unit 20 by a user U by way of uploading the medical image study MIS to the computing unit 20.

Optionally, supplemental information (or associate data) may be obtained for the medical image study MIS. The supplemental information may comprise medical text reports MTR, demographic information of the patient, a medical history of the patient, a diagnostic task to be performed based on the medical image study MIS or the like. Obtaining the supplemental information may comprise querying the medical information system 40 for supplementary information, e.g., based on the patient ID of the patient.

At step S15 a medical text report MTR is obtained. The medical text report MTR may relate to the medical image study MIS in the sense that the medical text report MTR describes findings derivable from the medical image study MIS. The medical text report MTR may be an already finalized report which has been previously signed-off by the user U or any other competent medical personnel. Such medical text report MTR may be stored in the medical information system 40 and may be automatically obtained by querying the medical information system 40.

According to some examples, the medical text report MTR may be a report the user U is currently in the process of creating. In other words, the medical text report MTR may also relate to a non-finalized report which the system 1 may analyze "on the fly" while being created by the user U. Such medical text report MTR may be created by the user U using the user interface 10 via which the user U may edit the medical text report MTR, e.g., by typing text or by dictating text into a speech-to-text module (not shown) of the user interface 10.

Step S15 is optional in the sense that the ensuing steps may also be performed if no medical text report MTR is available. In such cases, the subsequent processing may be based on other available supplementary information. Further, according to some examples, the medical text report MTR may also be comprised in the supplementary information.

At step S20, a context information CI is derived from the available information and data which may include the medical image study MIS, the medical text report MTR and/or any other supplementary information. Specifically, an accordingly configured function CI_FU may be applied to the available data which is configured to derive a meaningful context information CI therefrom.

According to some examples, determining the context information CI may comprise classifying the current case according to a number of predefined use-cases based on the available information. The use-case into which the current case is classified may then be the context information CI. According to some examples, the function CI_FU may be configured to perform the above classification. The use-cases may relate to the type of examination performed or the type of medical image study (e.g., CT or MR-study, with or without contrast agent, etc.), the suspected disease of the patient, the reason for the examination, the medical findings identified, the template used for the medical text report MTR, and so forth.

At optional sub-step S21, a medical finding of a given finding type is obtained. This may comprise identifying such a medical finding automatically in the available information and data. For instance, a medical finding may be identified in the medical text report MTR, for instance, based on an analysis of the text comprised in the medical text report MTR. Further, finding detection functions may be applied to medical image study MIS which detection functions are configured to identify medical findings in medical image data. Further, sub-step S21 may comprise identifying a medical finding based on the interactions of the user U with the system 1. Such user interactions may comprise the addition of a marking in a representation of the medical image study MIS shown in the user interface 10. Further, user interactions may also involve using a measurement tool to identify/measure the medical finding. According to further examples, the user input may also comprise activating an automated detection function configured to identify medical findings in medical image studies MIS.

At step S30, a template video script TVS is retrieved from the template database R_CVS which matches the context information CI. To this end, a lookup operation may be performed in the template database R_CVS for candidate template video scripts CVS corresponding to the context information CI.

According to some examples, the template database R_CVS may comprise a candidate template video script CVS per pre-defined use-case. Then, an association linking the candidate template video scripts CVS in the template database R_CVS with applicable use-cases may be used to identify the correct template video script TVS among the candidate template video scripts CVS based on the use-case of the current medical image study MIS.

According to some examples, the context information CI may be matched with the appropriate template video script TVS using an appropriately configured selection function TVS_FU.

At step S40, the medical video report MVR is rendered by executing the template video script TVS.

Here, the actual rendering process in the sense of transferring the image data comprised in the medica image study MIS into viewable images may be performed by a rendering function REN_FU. The rendering function REN_FU can implement various methods for visualizing a medical image study MIS (which may be a three-dimensional image data record), either individually or in combination. By way of example, the rendering function REN_FU can comprise a ray-casting module and/or a path-tracing module.

At step S50, the medical video report MVR is provided. This may, in particular, comprise making the medical video report MVR available for the human recipient HR. This may mean forwarding the medical video report MVR to the recipient interface 11 or holding the medical video report MVR available for download via the recipient interface 11. Further, step S50 may comprise providing the medical video report MVR to the user U for review and approval, e.g., in the user interface 10.

In Figure 4 a template video script TVS (or candidate template video script CVS) according to an embodiment is shown. Thereby, a template video script TVS, CVS may be seen as a recipe for creating a medical video report MVR for are certain use-case. In particular, the template video scripts TVS, CVS may comprise instructions for rendering a certain video-visualization and/or adding already existing video snippets or standard video sequences SEQ.

As can be seen from Figure 4, the template video scripts TVS, CVS may comprise different blocks or video template structures TVS each comprising instructions for providing a portion of the final medical video report MVR. According to some examples, the video template structures VTS may be respectively specific for a certain type of medical finding. Individual video template structures VTS may comprise instructions for optimally highlighting a certain medical finding for the human recipient HR. These instructions may include instructions to appropriately segment the medical image study MIS, to apply suited textures, use the right perspective, scene illumination, include markings, camera positions, animations, zoom levels and so forth. Further individual video template structure VTS may include instructions to append standard video sequences SEQ which may provide more general information for the human recipient HR and are independent form the image data. Such standard video sequences SEQ may relate to explanations regarding the diseases (i.e., the use-case), the examination performed for obtaining the medical image study MIS, or recommendations for the ensuing therapy.

Thus, in other words, a template video script TVS may script a sequence of standard videos and/or API calls to the rendering engine. According to a non-limiting example, individual video template structures VTS of an exemplary template video script TVS may look as follows in a pseudo-code representation:

| TVS | Instructions |
|---|---|
| TVS1 | appendVideo("Introduction CT Exam for medical finding of type AAA") |
| TVS2 | If existsFinding(AAA) then {"segment aorta", "display aorta", "zoom in", "highlight (segmentation object)", "overlay text 'aneurysm' at position (x,y)"} |
| TVS3 | If existsFinding(AAA) && AAA.size<=5cm then appendVideo("Recommendation AAA Surveillance") |
| TVS4 | If existsFinding(AAA) && AAA.size>5cm then appendVideo("Recommendation AAA Referral to Surgery") |

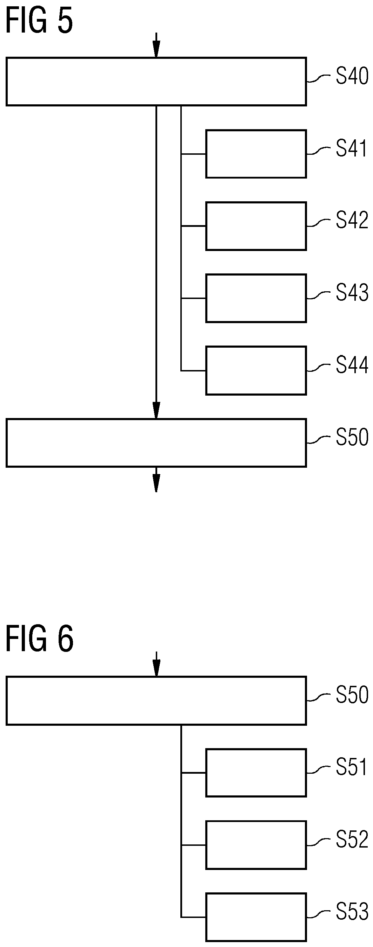

According to some examples, individual video template structures VTS may be activated for producing the medical video report MVR according to the circumstances of the case. Corresponding optional method steps are shown in Figure 5. Individual or all steps shown in Figure 5 may be carried out in step S40. The order of the steps does not necessarily correspond to the numbering of the steps but may also vary between different embodiments of the present invention. Further, individual steps or a sequence of steps may be repeated.

At step S41, individual video template structures VTS of the selected template video script TVS may be selectively activated or deactivated. While activated video template structures VTS are used for rendering the medical video report MVR, deactivated or not activated video template structures VTS are not used for rendering the medical video report MVR.

The activation of individual video template structures VTS may again depend on the circumstances of the case, i.e., the context information CI, the supplementary information, the medical text report MTR, the medical findings and, in particular, individual properties of the medical findings such as their size, as also shown in the above example.

At step S42 the activated video template structures VTS are executed, and the medical video report MVR is generated on that basis.

Further, at step S43, one or more standard video sequences SEQ may be selected, in particular, by activating a corresponding video template structure VTS. Again, the selection of a standard video sequence SEQ may be based on the circumstances of the case, i.e., the context information CI, the supplementary information, the medical text report MTR, the medical findings and, in particular, individual properties of the medical image study MIS such as the image modality used. At step S44, the selected standard video sequence(s) SEQ are retrieved from the standard video database R_SEQ and included in the medical video report MVR at the appropriate position (which may be determined by the position of the corresponding video template structure VTS in the template video script TVS) .

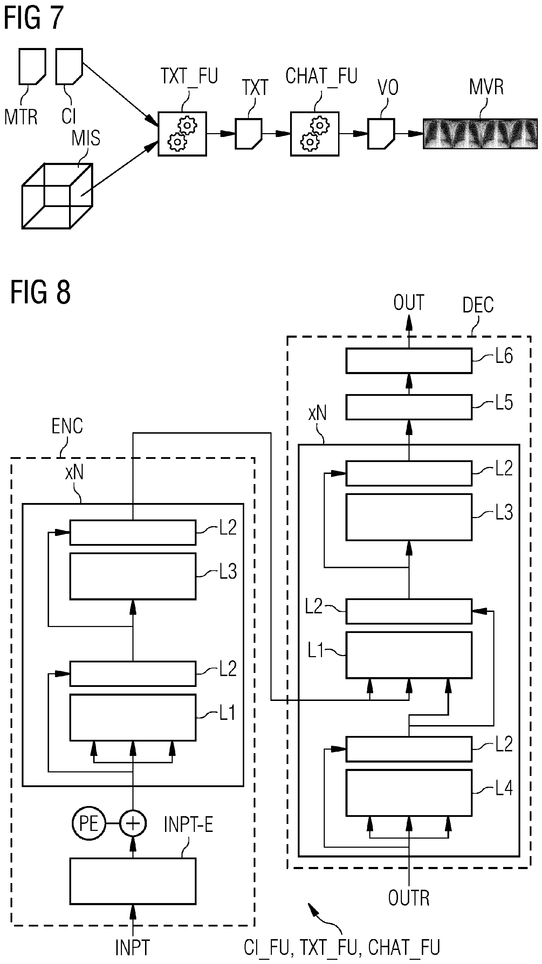

Optionally, the medical video report MVR may also comprise an audio track with a voice-over VO providing further explanations regarding the contents of the medical video report MVR. Figure 6 depicts a method for providing a voice-over VO according to an embodiment. Corresponding data streams are illustrated in Figure 7. The method comprises several steps. The order of the steps does not necessarily correspond to the numbering of the steps but may also vary between different embodiments of the present invention. Further, individual steps or a sequence of steps may be repeated.

At step S51, natural language text TXT is extracted/generated from the available information. The natural language TXT may summarize the relevant aspects of the case in text format but is not necessarily suited to be directly transferred into a voice-over VO. For instance, the text TXT may comprise redundancies, unsuited or complicated language, or may not have the adequate length for the medical video report MVR. Yet, the natural language text TXT may form the basis for the voice-over VO.

The natural language text TXT may be extracted using a text generation function TXT_FU (another expression may be mapping function) configured to turn (or map) medical findings and other relevant information into structured or unstructured text TXT. To this end, the text generation function TXT_FU may be configured to process medical image study MIS, the medical text report MTR, the context information CI, the medical findings detected, the video template structures VTS activated, and/or any other supplementary information.

At step S52, the text TXT generated in step S51 is brought into a version suited for the voice-over VO.

To this end, a chat function CHAT_FU may be provided which is configured to transcribe the text TXT into the version to be used for the voice-over VO. This may include shortening the text TXT, exchanging expressions in the text TXT, switching the language of the text TXT and so forth. According to some examples, the chat function CHAT_FU may be configured to simplify the text TXT and, in particular, replace technical terms with layperson explanations which are more readily understandable for the human recipient HR.

In this regard, the chat function CHAT_FU may be configured to take the specific requirements or constraints of the human recipient HR into consideration such as the language, the expertise level, and the like.

Further, the chat function CHAT_FU may be configured to take the medical video report MVR and, in particular, the activated video template structures VTS and the duration of the corresponding portions in the medical video report MVR into account.

At step S53, the voice-over VO is generated. This may include generating a corresponding soundtrack and including the soundtrack in the medial video report MVR.

Several functions which may be used for finally generating the medical video report MVR rely at least partially on the processing of natural language text TXT. In particular, this holds true for the context function CI_FU configured to determine the context information CI from the available information, the function TVS_FU configured to select the appropriate template video scripts TVS, the text generation function TXT_FU and the chat function CHAT_FU. According to some examples, one or more of these functions may be implemented as a machine learning model which is configured as a transformer network.

In Figure 8, a schematic representation of a transformer network according to an embodiment is shown. The transformer architecture follows an encoder-decoder structure and comprises an encoder ENC and a decoder DEC. In brief, the task of the encoder ENC is to map an input INPT to a sequence of continuous representations, which is then fed into a decoder DEC. The decoder DEC receives the output of the encoder ENC together with the decoder output OUTR at a previous iteration to generate an output OUT.

The encoder ENC of this embodiment may comprise of a stack of N=6 identical layers. For the sake of easy reference, only one layer xN is shown in the drawing. Further, N may also be set to different values and, in particular, to values greater than N=6 according to the respective task. Each layer xN of the encoder ENC comprises two sublayers L1 and L3. The first sublayer L1 implements a so-called multi-head self-attention mechanism. Specifically, the first sublayer L1 may be configured to determine how relevant a particular word is with regard to other words in the input INPT. This may be represented as an attention vector. To avoid any bias, multiple attention vectors per word may be generated and fed into a weighted average to compute the final attention vector of every word. The second sublayer L3 is a fully connected feed-forward network which may, for example, comprise two linear transformations with Rectified Linear Unit (ReLU) activation in between. The N=6 layers of the encoder ENC apply the same linear transformations to all the words in the input INPT, but each layer employs different weight and bias parameters to do so. Each sublayer L1, L3 is succeeded by a normalization layer L2, which normalizes the sum computed between the input fed into the respective sublayer L1, L3, and the output generated by the respective sublayer L1, L3 itself. In order to capture information about the relative positions of the words in the input INPT, positional encodings PE are generated based on input embeddings INPT-E prior to being fed into the layers xN. The positional encodings PE are of the same dimension as the input embeddings INPT-E and may be generated using sine and cosine functions of different frequencies. Then, the positional encodings PE may be simply summed to the input embeddings INPT-E in order to inject the positional information PE. Input embeddings INPT-E may be, as usual, a representation of each word in the input INPT, typically in the form of a real-valued vector that encodes the meaning of the word such that the words that are closer in the vector space are expected to be similar in meaning. According to some examples, a neural network may be used to generate the input embeddings INPT-E.

The decoder DEC of this embodiment may also comprise of a stack of N=6 identical layers xN each comprising three sublayers L4, L1, L3 which may be succeeded by a normalization layer L2 as explained in connection with the encoder ENC. For the sake of easy reference, only one layer xN of the decoder DEC is shown in the drawing. Further, N may also be set differently and, in particular, greater than N=6 according to the respective task. While the sublayers L1 and L3 of the decoder DEC correspond in their functionality to the respective sublayers L1 and L3 of the encoder ENC, sublayer L4 receives the previous output OUTR of the decoder DEC (optionally transformed into corresponding embeddings and augmented with positional information if the output is a sequence of words), and implements multi-head self-attention over it weighing how important individual elements of the previous output vector OUTR are. That followed, the values from the first sublayer L4 of the decoder DEC are input in the L1-sublayer of the decoder DEC. This sublayer L1 of the de-coder DEC implements a multi-head self-attention mechanism similar to the one implemented in the first sublayer L1 of the encoder ENC. On the decoder side, this multi-head mechanism receives the values from the previous decoder sublayer L4 and the output of the encoder ENC. This allows the decoder to attend to all the words in parallel. Like in encoder ENC part, the output of the L1 sublayers is passed into a feed-forward layer L2, which will make the output vectors form into something which is easily acceptable by another decoder block or a linear layer. After all layers xN of the decoder DEC have been processed, the intermediate result is fed into a linear layer L5 which may be another feed-forward layer. It is used to expand the dimensions into a format expected for computing the output vector OUT. That followed, the result is passed through a Softmax Layer L6, which transforms the result into an output which, as the case may be, may be the context information CI, the appropriate template video script TVS, the natural language text TXT or the text version for the voice-over VO.

Wherever meaningful, individual embodiments or their individual aspects and features can be combined or exchanged with one another without limiting or widening the scope of the present invention. Advantages which are described with respect to one embodiment of the present invention are, wherever applicable, also advantageous to other embodiments of the present invention. Independent of the grammatical term usage, individuals with male, female or other gender identities are included within the term.

## Claims

1. Computer-implemented method for providing a medical video report (MVR), the method comprising:
- receiving (S10) a medical image study (MIS) of a patient
- determining (S20), based on the medical image study (MIS), a context information (CI) pertaining to the medical image study (MIS),
- selecting (S30), based on the context information, a template video script (TVS) from a plurality of candidate template video scripts (CVS), each candidate template video script (CVS) encoding instructions for image processing steps of a medical image study (MIS) in order to generate a video relating to the medical image study (MIS) to a human recipient (HR),
- executing (S40) the selected template video script on the medical image study (MIS) so as to generate the medical video report (MVR),
- providing (S50) the medical video report (MVR).

2. Method according to claim 1, further comprising
- obtaining (S15) a medical text report (MTR) relating to the medical image study (MIS),
- wherein, in the step of determining (S20), the context information (CI) is extracted from on the medical text report (MTR) .

3. Method according to any one of the preceding claims, wherein
- the context information (CI) comprises an indication of a medical finding of a predefined finding type,
- the selected template video script (TVS) encodes instructions for image processing steps of a medical image study (MIS) in order to generate a video visualizing a medical finding of the predefined finding type for a human recipient (HR).

4. Method according to any one of the preceding claims, wherein
- the candidate template video scripts (CVS) respectively comprise a plurality of optionally activable video template structures (VTS) each encoding instructions for providing a portion of a medical video report (MVR) based on a medical image study (MIS),
- the step of executing (S40) comprises:
- activating (S41), based on the context information (CI), at least one of the video template structures (VTS) of the selected template video script (TVS), and
- generating (S42) the medical video report (MVR) based on executing the at least one activated video template structure (VTS).

5. Method according to claim 4, wherein
- the video template structures (VTS) of the selected template video script (TVS) comprise finding-specific video template structures (VTS) respectively with instructions for visualizing a medical finding of a predefined finding type,
- obtaining an indication of a medical finding of a given finding type based on the medical image study (MIS) and/or the context information (CI) and/or supplementary information associated to the medical image study (MIS), and
- the step of activating (S41) comprises activating, based on the given finding type, at least one finding-specific video template structure (VTS) of the selected template video script (TVS) the predefined finding type of which matches the given finding type.

6. Method according to claim 5, wherein the step (S21) of obtaining the at least one medical finding comprises,
- querying if the medical image study (MIS) indicates one or more medical findings of the predefined finding types of the video template structures (VTS) of the selected template video script (TVS).

7. Method according to any one of the preceding claims, wherein
- the selected template video script (TVS) comprises at least a video template structure (VTS) relating to a preexisting video sequence (SEQ),
- the step of executing (S40) comprises:
- retrieving (S43) the preexisting video sequence from a repository (R_SV) configured to store a plurality of preexisting video sequences (SEQ),
- including (S44) the preexisting video sequence (SEQ) in the medical video report (MVR).

8. Method according to any one of the preceding claims, further comprising:
- obtaining (S51) natural language text (TXT) from the context information (CI) and/or the medical image study (MIS),
- generating (S52) a voice-over (VO) based on the natural language text (TXT), and
- including (S53) the voice-over (VO) in the medical video report (MVR).

9. Method according to claim 8 in combination with claim 2, wherein
in the step (S51) of obtaining natural language text (TXT), the natural language text (TXT) is extracted from the medical text report (MTR).

10. Method according to any one of claims 8 or 9 in combination with claim 3, wherein
- the step (S51) of obtaining natural language text (TXT) comprises
- providing a mapping function configured to map medical findings onto structured text,
- generating the natural language text (TXT) by applying the mapping function to the medical finding.

11. Method according to any one of claims 8 to 10, wherein
- the step (S52) of generating the voice-over (VO) comprises
- providing a chat function (CHAT_FU) configured to transcribe natural language text (TXT) from a first version to a second version,
- applying the chat function (CHAT_FU) to the natural language text (TXT) so as to transcribe the natural language text into the second version, and,
- generating the voice-over (VO) based on the natural language text (TXT) of the second version.

12. Method according to any one of the preceding claims, wherein
- the medical image study (MIS) comprises a three-dimensional medical image data set, the selected template video script (TVS) encodes instructions for volumetric image rendering, in particular, implementing a path-tracing- or ray-casting-based rendering process, and
- the medical video report (MVR) comprises one or more images generated via volumetric image rendering.

13. System for providing a medical video report (MVR) comprising an interface unit configured to receive (S10) a medical image study (MIS) and a computing unit, the computing unit being configured to:
- determine (S20), based on the medical image study (MIS), a context information (CI) pertaining to the medical image study (MIS),
- select (S30), based on the context information, a template video script (TVS) from a plurality of candidate template video scripts (CVS), each candidate template video script (CVS) encoding instructions for image processing steps of a medical image study (MIS) in order to generate a video relating to the medical image study (MIS) for a human recipient (HR),
- execute (S40) the selected template video script (TVS) on the medical image study (MIS) so as to generate the medical video report (MVR), and
- provide (S50) the medical video report (MVR) via the interface unit.

14. Computer program product comprising program elements which induce a computing unit (20) of a system (1) to perform the steps according to the method of any one of claims 1 to 12, when the program elements are loaded into a memory of the computing unit (20).

15. Computer-readable medium on which program elements are stored that are readable and executable by a computing unit (20) of a system (1) to perform steps of the method according to any one of claims 1 to 12, when the program elements are executed by the computing unit (20).
